# EUROPEAN PATENT APPLICATION

(11) **EP 2 394 637 A1**
(43) Date of publication of application: **14.12.2011**
(21) Application number: 10460017.6
(22) Date of filing: 21.05.2010
(51) Int. Cl.: A61K 9/00, A61K 31/542, A61L 2/00

(54) **Process for obtaining sterile brinzolamide suspensions**

(71) Applicant: Zaklady Farmaceutyczne Polpharma SA, 83-200 Starogard Gdanski (PL)
(72) Inventor: Malgorzata, Stokrocka, 80-180 Gdansk (PL); Przemyslaw, Reszka, 83-200 Starogard Gdanski (PL)

(57) **Abstract**

The invention relates to methods of manufacturing pharmaceutical suspensions comprising brinzolamide sterilized by gamma irradiation or ethylene oxide, to compositions obtainable by this process and to the use of brinzolamide thus sterilized for the preparation of sterile ophthalmic composition. The invention further relates to pharmaceutical compositions of brinzolamide comprising less than 0.1% of chiral impurity of brinzolamide.

## Description

The invention relates to methods of manufacturing pharmaceutical suspensions comprising brinzolamide sterilized by gamma irradiation or ethylene oxide, to compositions obtainable by this process and to the use of brinzolamide thus sterilized for the preparation of sterile ophthalmic composition. The invention further relates to pharmaceutical compositions of brinzolamide comprising less than 0.1% of chiral impurity of brinzolamide.

### BACKGROUND OF THE INVENTION

Brinzolamide is a carbonic anhydrase inhibitor used to lower intraocular pressure in patients with ocular hypertension or open-angle glaucoma. The compound was disclosed in European patent EP 527801. International patent application WO 98/25620 teaches that conventional sterilization methods cannot be employed in the manufacture of suspensions comprising brinzolamide since the compound recrystallizes at autoclaving temperatures forming large needle-type crystals. According to WO 98/25620, also dry heat sterilization is not suitable since it causes melting of the material, whereas sterilization by ethylene oxide and gamma irradiation introduces unacceptable degradation products.

### SUMMARY OF THE INVENTION

Unexpectedly, it was found that contrary to the teaching of the prior art brinzolamide can be sterilized by ethylene oxide or gamma irradiation without obtaining degradation products in unacceptable for regulatory filings amounts. Furthermore, sterilization of brinzolamide according to the process of the invention results in obtaining less chiral impurity when compared to products manufactured by methods comprising applying autoclaving temperatures on brinzolamide.

According to a first aspect, the invention relates to a process for the manufacture of sterile ophthalmic suspensions comprising sterilization of brinzolamide by gamma irradiation or ethylene oxide. The process according to the present invention might further comprise adding to sterile brinzolamide at least one sterile solution comprising at least one pharmaceutically acceptable excipient. In a preferred embodiment, the process comprises the addition to sterile brinzolamide of a sterile solution comprising at least one surfactant. In a more preferred embodiment of the invention, at least one surfactant, at least one isotonicity agent, at least one viscosity enhancing agent and at least one preservative are added in the form of at least one sterile solution to the sterile brinzolamide.

A further aspect of the invention relates to pharmaceutical composition comprising brinzolamide obtainable by the process according to the first aspect of the invention.

A further aspect of the invention relates to the use of brinzolamide sterilized by gamma irradiation or ethylene oxide for the manufacture of ophthalmic compositions.

A further aspect of the invention relates to pharmaceutically acceptable suspensions comprising brinzolamide and at least one pharmaceutically acceptable excipient containing less than 0.1% of chiral impurity of brinzolamide, i.e. (S)-4-(ethylamino)-3,4-dihydro-2-(3-metoxypropyl)-2H-thieno [3,2e]-1,2-thiazine-6-sulfonamide-1,1-dioxide. Percentage of impurity is referred to the amount of brinzolamide (w/w %). The process of the invention enables the preparation of pharmaceutical suspensions with lower amounts of impurities than the methods of sterilization of brinzolamide known in the art, which is always desirable from a patient perspective.

### DETAILED DESCRIPTION OF THE INVENTION

The term "brinzolamide" is to be understood in the present invention as (R)-4-(ethylamino)-3,4-dihydro-2-(3-metoxypropyl)-2H-thieno [3,2e]-1,2-thiazine-6-sulfonamide-1,1-dioxide or its pharmaceutically acceptable salts.

The sterilization of brinzolamide by gamma irradiation or ethylene oxide according to the invention is performed by methods known to the person skilled in art. Particularly, sterilization by gamma irradiation is performed employing any pharmaceutically acceptable dose of gamma irradiation, preferably the dose of either 5, 10, 15 or 25 kGy, most preferably 15 kGy, whereas sterilization by ethylene oxide is performed preferably using pure ethylene oxide.

The pharmaceutical suspension comprising brinzolamide sterilized by gamma irradiation or ethylene oxide and pharmaceutically acceptable excipients according to the invention is prepared by methods known to the person skilled in art. The process of obtaining pharmaceutical suspension according to the invention can further comprise adding at least one sterile solution comprising at least one pharmaceutically acceptable excipient to brinzolamide sterilized by gamma irradiation or ethylene oxide. In a preferred embodiment of the invention, at least one surfactant, at least one isotonicity agent, at least one viscosity enhancing agent and at least one preservative are added in the form of at least one sterile solution to the sterile brinzolamide. The sterile excipient solutions according to the invention can be obtained by dissolving the excipients in water and sterilizing thus obtained solutions by methods known from the state of art, preferably by autoclaving or sterile filtration.

In a most preferred embodiment of the invention, the process of obtaining pharmaceutical suspension comprising brinzolamide employs the following steps:
1. Sterilization of brinzolamide by gamma irradiation or ethylene oxide.
2. Preparation of sterile brinzolamide suspension by adding a sterile solution comprising at least one surfactant to the sterile brinzolamide and mixing until homogeneity is obtained.
3. Preparation of a sterile solution comprising at least one viscosity enhancing agent,
4. Preparation of a sterile solution comprising at least one isotonicity agent, at least one preservative and, optionally, additional excipients.
5. Preparation of drug product, i.e. final brinzolamide suspension, by mixing sterile brinzolamide suspension of step 2, sterile solution comprising at least one viscosity enhancing agent of step 3 and sterile excipient solution of step 4 until homogeneity is obtained and adjusting pH to approximately 7,5 with sterile NaOH solution.

Surfactants according to preferred embodiments of the invention might be pharmaceutically acceptable wetting agents commonly used in ophthalmic suspensions such as, but not limited to, Tyloxapol, i.e. 4-(1,1,3,3-tetramethylbutyl)phenol polymer with formaldehyde and oxirane, commercially available from Sterling Co., Triton X-100, i.e. α-[4-(1,1,3,3-tetramethylbutyl)phenyl]-ω-hydroxypoly oxy-1,2-ethane diyl, commercially available from Rohm and Haas Corp., sodium lauryl sulfate, docusate sodium, polyoxyalkyl ethers, polyoxylalkyl phenyl ethers, polyoxy hydrogenated castor oil, polyoxy sorbitan esters, sorbitan esters, preferably tyloxapol.

Isotonicity agents according to the invention could be selected from the group comprising dextrose, glycerin, mannitol, potassium chloride, sodium chloride.

Viscosity enhancing agents according to the present invention could be preferably selected from the group comprising carbomer, poloxamer, polyvinyl alcohol, povidone, polyethylene oxide, carboxymethylcellulose calcium, carboxymethylcellulose sodium, hydroxyethyl cellulose, hydroxypropyl cellulose, hydroxymethyl cellulose, hydroxypropyl methylcellulose, methylcellulose.

Preservatives according to the invention could be preferably selected from the group comprising benzalkonium chloride, benzoic acid, benzyl alcohol, butylparaben, propylparaben, methylparaben, chlorobutanol, phenoxyethanol.

The invention relates also to pharmaceutical compositions comprising brinzolamide and at least one pharmaceutically acceptable excipient characterized in having less chiral impurity when compared to products manufactured by methods comprising applying autoclaving temperatures on brinzolamide.

In an experiment, the pharmaceutical suspension comprising pharmaceutically acceptable excipients as given in Example 3 was reproduced four times comprising respectively:
1. brinzolamide sterilized by autoclaving,
2. brinzolamide not sterilized,
3. brinzolamide sterilized by gamma irradiation,
4. brinzolamide sterilized by ethylene oxide,
and the amount of chiral impurity of brinzolamide was measured.

The below table presents the relevant amounts of chiral impurity of brinzolamide, , i.e. (S)-4-(ethylamino)-3,4-dihydro-2-(3-metoxypropyl)-2H-thieno [3,2e]-1,2-thiazine-6-sulfonamide-1,1-dioxide, by weight of brinzolamide measured directly after preparing of the suspension (at t=0).

| Pharmaceutical suspension comprising pharmaceutically acceptable excipients as given in Example 3 and: | Amount of chiral impurity of Brinzolamide measured at t=0 [%] |
|---|---|
| brinzolamide sterilized by autoclaving | 0,32 |
| brinzolamide not sterilized | below 0,1 |
| brinzolamide sterilized by gamma irradiation | below 0,1 |
| brinzolamide sterilized by ethylene oxide | below 0,1 |

The above results obtained in the experiment prove that the sterilization methods of the invention introduce less chiral impurity of brinzolamide to the product compared to sterilization of brinzolamide by autoclaving.

The following examples illustrate various aspects of the present invention. They are not to be construed to limit the claims in any manner whatsoever.

### EXAMPLES

### Example 1

Process of sterilization of brinzolamide by ethylene oxide.

20 g of brinzolamide was treated with pure ethylene oxide for 200 minutes under reduced pressure of 612 mBar in Sterimed 2 sterilization device. The degasation time was 30 hours.

Biological testing confirmed that brinzolamide obtained in the process was sterile.

### Example 2

Process of sterilization of brinzolamide by gamma irradiation.

50 g of brinzolamide was treated with gamma irradiation dose of 15 kGY for 3,4 seconds in dosimeter ECB.

Biological testing confirmed that brinzolamide obtained in the process was sterile.

### Example 3

Manufacturing process for 2000 g of sterile pharmaceutical suspension comprising brinzolamide sterilized by ethylene oxide and the following auxiliary ingredients:

| Ingredient | Amount [g/100g] | Amount [mg/g] | Quantity per batch [g] |
|---|---|---|---|
| Brinzolamide | 1,000 | 10,000 | 20,00 |
| Tyloxapol | 0,025 | 0,250 | 0,50 |
| Carbopol 974P | 0,400 | 4,000 | 8,00 |
| Mannitol | 3,300 | 33,000 | 66,00 |
| Sodium Chloride | 0,250 | 2,500 | 5,00 |
| Disodium EDTA | 0,010 | 0,100 | 0,20 |
| Benzalconium Chloride (50% BAC) | 0,020 | 0,200 | 0,40 |
| 1N NaOH approx. | q.s | q.s. | q.s. |
| Aqua purified | ad 100,000 | ad 1,000 | ad 2000,00 |

### Step 1: Preparation of sterile brinzolamide suspension.

Approximately 80 g of purified water and 0,50 g of tyloxapol are added into a mixer and stirred until homogeneous solution is obtained. The solution is passed through 40 µm filter and autoclaved. 20 g of brinzolamide sterilized as presented in example 1 is added to thus obtained sterile tyloxapol solution and stirred until homogeneous suspension is obtained.

### Step 2: Preparation of first sterile excipient solution.

Approximately 720 g of pure water and 8,00 g of Carbopol 974P are added into a mixer and stirred until homogeneous solution is obtained. Thus obtained solution is passed through 40 µm filter and autoclaved.

### Step 3: Preparation of second sterile excipient solution.

Approximately 900 g of pure water, 66 g of mannitol, 5 g of sodium chloride, 0,2 g of disodium EDTA and 0,4 g of benzalconium chloride (50% BAC) are introduced into a mixer and stirred until homogeneous solution is obtained. The solution is passed through 0,22 µm filter to provide sterilization.

### Step 4: Preparation of drug product, i.e. final sterile brinzolamide suspension.

The sterile brinzolamide suspension of step 1, first sterile excipient solution of step 2 and second sterile excipient solution of step 3 are transferred to a mixer and stirred. After complete mixing pH is adjusted to 7,5 with sterile NaOH solution.

### Example 4

Manufacturing process for 5000 g of sterile pharmaceutical suspension comprising brinzolamide sterilized by gamma irradiation and the following auxiliary ingredients:

| Ingredient | Amount [g/100g] | Amount [mg/g] | Quantity per batch [g] |
|---|---|---|---|
| Brinzolamide | 1,000 | 10,000 | 50,00 |
| Tyloxapol | 0,025 | 0,250 | 1,25 |
| Carbopol 974P | 0,400 | 4,000 | 20,00 |
| Mannitol | 3,300 | 33,000 | 165,00 |
| Sodium Chloride | 0,250 | 2,500 | 12,50 |
| Disodium EDTA | 0,010 | 0,100 | 0,50 |
| Benzalconium Chloride (50% BAC) | 0,020 | 0,200 | 1,00 |
| 1N NaOH approx. | q.s | q.s. | q.s. |
| Aqua purified | ad 100,000 | ad 1,000 | ad 5000,00 |

### Step 1: Preparation of sterile brinzolamide suspension.

Approximately 200 g of purified water and 1,25 g of tyloxapol are added into a mixer and stirred until homogeneous solution is obtained. The solution is passed through 40 µm filter and autoclaved. 50 g of brinzolamide sterilized as presented in example 2 is added to thus obtained sterile tyloxapol solution and stirred until homogeneous suspension is obtained.

### Step 2: Preparation of first sterile excipient solution.

Approximately 1800 g of pure water and 20,00 g of Carbopol 974P are added into a mixer and stirred until homogeneous solution is obtained. Thus obtained solution is passed through 40 µm filter and autoclaved.

### Step 3: Preparation of second sterile excipient solution.

Approximately 2200 g of pure water, 165 g of mannitol, 12,50 g of sodium chloride, 0,5 g of disodium EDTA and 1,0 g of benzalconium chloride (50% BAC) are introduced into a mixer and stirred until homogeneous solution is obtained. The solution is passed through 0,22 µm filter to provide sterilization.

### Step 4: Preparation of drug product, i.e. final sterile brinzolamide suspension.

The sterile brinzolamide suspension of step 1, first sterile excipient solution of step 2 and second sterile excipient solution of step 3 are transferred to a mixer and stirred. After complete mixing pH is adjusted to 7,5 with sterile NaOH solution.

## Claims

1. A process for the manufacture of sterile ophthalmic suspensions comprising brinzolamide, **characterized in that** the process comprises the step of sterilization of brinzolamide by gamma irradiation or ethylene oxide.

2. The process according to claim 1, wherein the process further comprises the addition to sterile brinzolamide of one or more sterile solutions comprising at least one pharmaceutically acceptable excipient.

3. The process according to claim 2, wherein the process comprises the addition to sterile brinzolamide a sterile solution comprising at least one surfactant.

4. The process according to claims 2 or 3, wherein the one or more sterile solutions comprise at least one surfactant, at least one isotonicity agent, at least one viscosity enhancing agent and at least one preservative.

5. The process according to claims 3 or 4, wherein the surfactant is selected from the group comprising tyloxapol, Triton X-100, sodium lauryl sulfate, docusate sodium, polyoxyalkyl ethers, polyoxylalkyl phenyl ethers, polyoxy hydrogenated castor oil, polyoxy sorbitan esters, sorbitan esters and mixtures thereof, preferably tyloxapol.

6. The process according to claims 4 or 5, wherein the viscosity enhancing agent is selected from the group comprising carbomer, poloxamer, polyvinyl alcohol, povidone, polyethylene oxide, carboxymethylcellulose calcium, carboxymethylcellulose sodium, hydroxyethyl cellulose, hydroxypropyl cellulose, hydroxymethyl cellulose, hydroxypropyl methylcellulose, methylcellulose and mixtures thereof.

7. The process according to claims 4 to 6, wherein the isotonicity agent is selected from the group comprising dextrose, glycerin, mannitol, potassium chloride, sodium chloride and mixtures thereof.

8. The process according to claim 4 to 7, wherein the preservative is selected from the group comprising benzalkonium chloride, benzoic acid, benzyl alcohol, butylparaben, propylparaben, methylparaben, chlorobutanol, phenoxyethanol and mixtures thereof.

9. Pharmaceutical composition comprising brinzolamide obtainable by the process of claims 1 to 8.

10. Use of brinzolamide sterilized by gamma irradiation or ethylene oxide for the manufacture of ophthalmic compositions.

11. Pharmaceutical composition comprising brinzolamide and at least one pharmaceutically acceptable excipient, **characterized in that** the composition comprises less than 0.1% of (S)-4-(ethylamino)-3,4-dihydro-2-(3-metoxypropyl)-2H-thieno [3,2e]-1,2-thiazine-6-sulfonamide 1,1 dioxide by weight of brinzolamide.
